Europäisches Patentamt

(19) European Patent Office

Office européen des brevets

(11) Publication number : **0 141 783 B2**

## (12) NEW EUROPEAN PATENT SPECIFICATION

(45) Date of publication of the new patent
specification : **16.06.93 Bulletin 93/24**

(21) Application number : **84810516.9**

(22) Date of filing : **25.10.84**

(51) Int. Cl.⁵ : **A61K 39/395,** C12N 5/00

(54) **Immune response to tumours and viruses induced by anti-idiotype antibodies.**

Divisional application 88102108 filed on
12.02.88.

(30) Priority : **07.11.83 US 549505**
**07.11.83 US 549506**

(43) Date of publication of application :
**15.05.85 Bulletin 85/20**

(45) Publication of the grant of the patent :
**09.08.89 Bulletin 89/32**

(45) Mention of the opposition decision :
**16.06.93 Bulletin 93/24**

(84) Designated Contracting States :
**AT BE CH DE FR GB IT LI LU NL SE**

(56) References cited :
**EP-A- 0 110 706**
**THE JOURNAL OF IMMUNOLOGY, vol. 133, no.
1, July 1984, pp. 495-501 (US), K. THIELEMANS
et al.: "Strategies for production of monoc-
lonal anti-idiotype antibodies against human
B cell lymphomas"**
**Proc. NATL. ACAD. SCI., USA, vol. 81, pp.
2864-2867, May 1984, (US), Immunology, G.T.
NEPOM et al.: "Induction of immunity to a
human tumor marker by in vivo administration
of antiidiotypic antibodies in mice"**
**PROC. NATL. ACAD. SCI., USA, vol. 81, pp.
216-219, January 1984 (US), Immunology, H.
KOPROWSKI et al.: "Human antiidiotype anti-
bodies in cancer patients: Is the modulation of
the immune response beneficial for the
patient?"**
**BIOLOGICAL ABSTRACTS, vol. 74, no. 1, 1982,
p. 345, ref.no. 3301, A. NISONOFF et al.:
"Implications of the presence of an internal
image of the antigen in antiidiotype anti-
bodies: Possible application to vaccine pro-
duction"**
**BIOLOGICAL ABSTRACTS, vol. 73, no. 5, 1982,
p. 3306, ref.no. 32091, J. Gheuens et al.:
"Idiotypes and biological activity of murine
monoclonal antibodies against the
hemagglutinin of measles virus"**
**THE JOURNAL OF IMMUNOLOGY, vol. 121, no.
6, December 1978, pp. 2358-2362 (US), G.
HAUGHTON et al.: "Antigen-induced murine B
cell lymphomas"**

(73) Proprietor : **THE WISTAR INSTITUTE
36th Street at Spruce
Philadelphia Pennsylvania 19104 (US)**

(72) Inventor : **DeFreitas, Elaine
731 Newtown Road
Villanova Pennsylvania 19085 (US)**
Inventor : **Herlyn, Dorothee
1223 Knox Road
Wynnewood Pennsylvania 19096 (US)**
Inventor : **Koprowski, Hilary
334 Fairhill Road
Wynnewood Pennsylvania 19096 (US)**
Inventor : **Reagan, Kevin
21 South Cliff Drive
Wycliffe Delaware 19809 (US)**
Inventor : **Wiktor, Tadeusz
9 Downs Circle
Wynnewood Pennsylvania 19096 (US)**

(74) Representative : **Bannerman, David Gardner et
al
Withers & Rogers 4 Dyer's Buildings Holborn
London, EC1N 2JT (GB)**

EP 0 141 783 B2

(56) References cited :
PROC. NATL. ACAD. SCI., USA, vol. 79, pp. 6651-6655, November 1982, (US), Immunology, D. KOZBOR et al.: "Human hybridomas constructed with antigen-specific Epstein-Barr virus-transformed cell lines"
NATURE, vol. 303, June 16, 1983, pp. 627-629, J.W. FORSTROM et al.: "Immunization to a syngeneic sarcoma by a monoclonal auto-anti-idiotypic antibody"
Binz et al. Int. Journal of Cancer vol. 29 pp 417-423 (1982)
Roitt et al. The Lancet 1981. pp. 1041-1045
Nisonoff & Lamoyi. Clinical Immunology Immunopathology.vol.21 pp. 397-401
Eichmann. "Anti-bodies in human diagnosis & therapy" (ed. Haber & Krause) Raven press. s. 299-302. 1977
Sacks et al. Journal of experimental medicine.vo. 155 pp. 1108-119 (1982)
Jerne et al. The EMBO Journal vol.1 pp 243-247 (1982)
Miller et al. Journal of medicine vol. 306 pp 517-522 (1982)
Hatzubai et al. Journal of immunology vol. 126. pp 2397-2402(1981)
Sege et al. PNAS. USA vol. 75 pp.2443-2447 (1978)

## Description

Technical field

The present invention is directed to the induction of immunological responses to antigens, in particular tumors. More specifically the present invention is directed to the use of anti-idiotype antibodies to induce such an immunological response, as well as the antibodies and cell lines that produce them.

Background of the invention

The sqeuence of amino acids in the variable regions of both heavy ($V_H$) and light ($V_L$) chains of immunoglobulin (Ig) produces a conformation in the antigen binding site (*i.e.*, parotope) allowing interaction of that antibody with a specific antigen. Injection of Ig into a heterologous host animal will give rise to anti-xenotypic (specific for species), anti-isotypic (specific for Ig class), and anti-idiotypic (specific for antibody variable region) antibodies. Two functional classes of anti-idiotypic antibodies can exist, one of which reacts with the parotope, and another which reacts with the $V_H$ and/or $V_L$ framework (framework determinants). *See generally,* Geha, (1981) *N. Engl. J. Med.* 305: 25-28; Jerne, (1974) *Ann. Immunol. (Paris) 125C*: 373-389.

Summary of the invention

It is an object of the present invention to provide a method of inducing an immunological response to tumors in particular, solid tumors.

It is also an object of the present invention to employ anti-idiotype antibodies to produce such an immunological response.

Yet another object of the present invention is to provide monocloncal anti-idiotype antibodies, and immortal B lymphocyte sources for such antibodies, that are useful in the induction of immunological responses to tumors, particularly solid tumors.

These and other objects of the present invention are achieved by one or more of the following embodiments.

In one embodiment, the present invention provides a method of inducing an immunological response to a tumor comprising:

(a) providing an anti-idiotype antibody, an epitope identified by said anti-idiotype anitbody being the parotope of an anti-tumor antibody; and

(b) stimulating in a subject the production of anti-(anti-idiotype) antibody that identifies an epitope on a tumor cell by administering said anti-idiotype antibody to said human.

The present invention also provides polyclonal anti-idiotype antibodies, an epitope identified by said anti-idiotype antibodies being the parotope of an anti-tumor antibody, substantially free of anti-isotypic antibodies.

In another embodiment, the present invention provides an immortal B lymphocyte that produces an anti-idiotype antibody, an epitope identified by said anti-idiotype antibody being the parotope of an anti-tumor antibody. The present invention also provides the monoclonal antibodies produced by the above immortal B lymphocyte substantially free of other antibodies.

Suitable tumors are solid tumors such as solid gastrointestinal tumors. On the administration of the anti-idiotype antibodies in accordance with the invention, the subject, in particular mammal, more particularly human, is stimulated to produce anti-(anti-idiotype) antibody that identifies an epitope on a tumor cell.

Detailed description of the invention

The present invention provides a unique approach to cancer therapy. Traditionally approaches in tumor therapy have involved administering anti-tumor antibodies (*i.e.*, antibodies that identify an epitope on a solid tumor cell) to patients in an effort to destroy the tumor. Applicants, however, have discovered that an immunological response to a patient's own tumors can be induced with an antibody that is anti-idiotypic to an antibody that recognizes a tumor. The induction of this immunological rseponse has utility as a therapeutic, and preventative treatment.

Although applicants do not wish to be bound by any particular theory of operability, it is believed that the observed immunological response achieved by the present invention is attributable to an interaction between the anti-idiotype antibody molecules and the human patient's immune system. The idiotypic (*i.e.*, variable) region of the anti-idiotype antibody molecule contains antigenic determinants (*i.e.*, epitopes) seen as an antigen by the subject. This induces the production of anti-(anti-idiotype) antibodies by the subject. Within this set of anti-(anti-idiotype) antibodies are those that are directly complimentary to the parotope of the anti-idiotype antibody. It is further believed that the parotope of the anti-idiotype antibody presents an "internal" image of the tumor cell identified (*i.e.*, selectively bound) by the idiotype antibody and, therefore, the anti-(anti-idiotype) antibodies will also bind the tumor. In effect, the present method induces an immunological response to the tumor by presenting an antigen (the parotope of the anti-idiotype antibody) which is essentially indistinguishable from the tumor to a portion of the patient's resulting antibodies.

Surprisingly, the above method is an effective procedure for controlling tumor growth. Furthermore, it has several advantages over the more traditional approaches. First, much less foreign antibody need be administered to a patient. Second, the patient's anti-idiotype response is beneficial rather detrimental to the intended effect. Third, the patient's own antibodies are the anti-tumor antibodies, thus eliminating the necessity of repeated administration of exogenous anti-tumor antibodies. Other advantages will be readily apparent to those skilled in the art.

The idiotype of an antibody is defined by individually distinctive antigenic determinants in the variable or idiotypic region of the antibody molecule. A portion of these idiotypic determinants will be on or closely associated with the parotope of the antibody, while others will be in the framework of the variable region. While each antibody has its own idiotype, particular antibodies will be referred to below by the following terms. "Idiotype antibody" or "Id Ab" refers to an anti-tumor antibody (*i.e.*, the epitope identified by the idiotype antibody is on a cell of a tumor. "Anti-idiotype antibody" or "anti-Id Ab" refers to an antibody which identifies an epitope in the variable region of an idiotype antibody. A portion of such antibodies will identify an epitope that is the parotope of the idiotype antibody, thus presenting an "internal" image of the epitope identified by the idiotype antibody on the tumor cell. "Anti-(anti-idiotype) antibody" or "anti-(anti-Id)Ab" is an antibody that identifies an epitope in the variable region of the anti-idiotype antibody. A portion of the anti-(anti-idiotype) antibodies will identify an epitope that corresponds to (i) the parotope of the anti-idiotype antibody, and (ii) an epitope on a tumor cell.

As stated below, the method of the present invention contemplates administering anti-idiotype antibody to a host. The anti-idiotype antibody is administered to the host in any physiologically suitable carrier (*e.g.*, sterile, pyrogen-free physiological saline), the formulations of which are within the skill of the art. The selection of carrier is not critical and the antibody can be administered by any method that introduces the antibody into the circulatory system (*e.g.*, intravenous, intramuscular or subcutaneous injection).

The host may be any mammal, most commonly a human. The amount of antibody administered to a host can vary widely dependent, for example, upon the particular antibody employed and the patient inoculated. It is only necessary that sufficient anti-idiotype antibody be administered to stimulate the production of anti-(anti-idiotype) antibodies by the patient's immune system. The amounts of antibody employed, however, need not be very great because only very small amounts are necessary to induce an immunological response. In many cases, a dosage of antibody within the range of a few micrograms to a few milligrams should be sufficient, (*e.g.*, about 50-200 µg to about 1-5 mg). The determination of an appropriate dosage is readily within the skill of the art.

In one embodiment, the present invention contemplates administering a formulation containing anti-idiotype antibody to a human patient to produce an immunological response to a tumor *e.g.* a solid tumor (*i.e.* a solid mass of malignant cells such as produced by carcinomas, sarcomas, melanomas etc., as opposed to disperse, circulating malignant cells such as leukemias). In a preferred embodiment, the tumor is a gastrointestinal tumor. As defined above, a subclass of the anti-idiotype antibody selectively binds to (*i.e.*, identifies) the parotope of an anti-tumor antibody (the idiotype antibody). Such an antibody recognises an epitope that is the parotope of the corresponding idiotype antibody.

Anti-idiotype antibodies, which present internal images of the tumor or virus antigen, can be distinguished from anti-idiotype antibodies that recognize framework determinants in the variable region of the idiotype antibody by any of several methods. One method of identifying the desired anti-idiotype antibodies is a competitive binding assay between the tumor or viral antigen (or hapten if available), the idiotype antibody and the anti-idiotype antibody. If the antigen blocks binding of the anti-idiotype antibody to the idiotype antibody, the epitope identified by the anti-idiotype antibody is closely associated with the idiotype antibody's parotope. Another test is to determine if anti-sera to the anti-idiotype antibody is also anti-tumor. These and other methods of identifying the appropriate anti-idiotype antibody are within the skill of the art. In the formulation administered to a host, the inclusion of anti-idiotype antibodies directed to framework determinants along with the subclass directed to the idiotype antibody's parotope is acceptable. It is only necessary that the formulation contain the subclass directed to the idiotype antibody's parotope.

The anti-idiotype antibody employed can be homologous or heterologous to the host. The preferred antibody for a human is however a human antibody to minimize immunological response to the constant region to the antibody molecule. However, since relatively small doses of anti-idiotype antibody are required in the present invention, heterologous antibody can be employed (*e.g.,* mouse, rat, goat, rabbit, etc.). In the absence of any serious reaction to heterologous anti-idiotype antibody, however, such antibody may be preferred due to ease and cost of preparation. Furthermore, polyclonal anti-idiotype antibodies can be employed as well as monoclonal anti-idiotype antibodies.

Polyclonal anti-idiotype antibody can be prepared by conventional methods known in the art. For example, polyclonal anti-Id Ab can be produced by immunizing an animal with a monoclonal anti-tumor antibody (*i.e.,* Id Ab). The immunized animal will produce anti-Id Ab. A subclass of this anti-idiotype antibody in the anti-sera will identify an epitope that is the parotope of the anti-tumor antibody.

Anti-sera collected from the animal can be purified, for example, by sequential absorption with (i) an immobilized antibody of the same isotype as the monoclonal Id Ab, but different idiotype, to remove anti-isotypic antibodies from the anti-sera, and (ii) the immobilized monoclonal Id Ab to remove the anti-Id Ab, a subclass of which will present internal images of the tumor or viral antigen. The anti-Id Ab can then be eluted from the bound monoclonal anti-tumor or viral antibody to provide a solution substantially free of anti-isotype antibodies. This solution can then be tested for the presence of anti-Id Ab that identifies the parotope of the Id Ab.

Monocloncal anti-idiotype antibodies substantially free of other antibodies can be isolated from the supernatant of substantially pure cultures of immortal B lymphocytes. The term "immortal B lymphocyte" encompasses any relatively stable, continuous antibody-producing cell that can be maintained in culture for several months (preferably indefinitely), such as hybridomas (somatic cell hybrids of normal and malignant lymphocytes) and normal lymphocytes transformed by virus (*e.g.,* Epstein-Barr virus) or oncogenic DNA. The production of immortal B lymphocytes from normal B lymphocytes that product anti-idiotype antibody is within the skill of the art. *See. e.g., Monocloncal Antibodies* (R. H. Kennett, T. J. McKearn & K. B. Bechtol 1980); M. Schreier et al., Hybridoma Techniques (Cold Spring Harbor Laboratory (1980); *Monoclonal Antibodies and T-Cell Hybridomas* (G. J. Hammerling, U. Hammerling & J. F. Kearney 1981); Kozbor et al., (1982) *Proc. Natl. Acad. Sci. U.S.A. 79*: 6651-6655; Jonak et al., (1983) *Hybridoma* 2:124; *Monoclonal Antibodies and Functional Cell Lines* (R. H. Kennett, K. B. Bechtol & T. J. McKearn 1983); Kozbor et al., (1983) *Immunology Today 4*: 72-79.

Normal B lymphocytes producing anti-Id Ab and suitable for the production of an immortal B lymphocyte can be provided by various methods within the skill of the art. For example, an animal, such as a rat or mouse, can be immunized with a monoclonal anti-tumor antibody and B lymphocytes producing anti-Id Ab recovered from the animal's spleen. Human B lymphocytes producing anti-Id Ab can be obtained by immunizing a patient with monoclonal anti-tumor antibody, collecting peripheral blood lymphocytes from the patient, and then inducing *in vitro* the growth of B lymphocytes producing anti-Id Ab by stimulating the culture with the monoclonal anti-tumor antibody. *See, e.g.,* DeFreites et al., (1982) *Proc. Natl. Acad. Sci. U.S.A. 79*: 6646-6650. The animal or human B lymphocytes producing anti-Id Ab can thus be recovered and immortalized by those of skill in the art. Of course it is understood that those lymphocytes producing anti-Id Ab that present internal images of the tumor cell should be distinguished from B lymphocytes producing anti-Id Ab directed to framework determinants in the idiotypic region.

The method of the present invention as applied to tumors can also be practiced in conjunction with other methods of inducing immunological responses to tumors such as the use of viral oncolysate vaccines as described in U.S. Patent 4,108,983.

The following examples are illustrative of the present invention and are not intended to limit its scope.

Example 1

Monoclonal idiotype antibodies

The following experiments employed mouse monoclonal antibodies 17-1A, $C_42032$ and $C_41472$, which bind to human gastrointestinal cancer cells, and are described in Herlyn et al., (1979) *Proc. Natl. Acad. Sci. U.S.A. 76*: 1438-1442 and Koprowski, "Monoclonal Antibodies *In Vivo,"* in *Monoclonal Antibodies in Cancer: Proceedings of the Fourth Armand Hammer Cancer Symposium* (B. Boss, R. Langman, I. Trowbridge & Dulbecco 1983). Monoclonal antibody (MAb) $C_42032$ has specificity for colorectal carcinoma (CRC)-associated antigen(s) of $M_r$ 180, 160, 50 and 40 K. MAb $C_41472$ (IgG2a) has specificity for CRC-associated antigen $M_r$ 50 K. The MAb A5C3 against hepatitis virus was also employed and is described in Wands et al., (1981) *Proc. Natl. Acad. Sci. U.S.A. 78*: 1214-1218. MAb's 17-1A (IgG2A, kappa light chain) and $C_42032$ (IgG2a kappa light chain) were purified from ascites obtained from hybridoma-bearing mice by affinity chromatography on protein A-sepharose column (Pharmacia, Piscataway, NJ) as described by Ey et al., (1978) *Immunochemistry 15*: 429-436.

Patients

All patients had metastatic or recurrent gastrointestinal adenocarcinoma and were injected systematically with one dose of a purified sterile, pyrogen-free preparation of MAb 17-1A concentrated from ascites fluid of Balb/c mice per the method disclosed in Sears et al., (1982) *Lancet* 762-765. Of nine patients who received 192 mg or less of MAb 17-1A seven developed anti-mouse globulin antibodies. Of the 20 patients who received 200-1000 mg of monoclonal antibody, three developed anti-mouse globulin antibodies. Sera of three patients of the first group who developed anti-mouse globulin molecules (patient Nos. 07, 08 and 09) and of two patients of the second group (Nos. 14 and 23), were either screened or processed for isolation of anti-idiotype antibodies. Sera used for isolation of anti-idiotype antibodies from subject Nos. 07, 08 and 23 were obtained at the time when all three showed the highest concentration of anti-mouse globulin antibodies. Patient No. 08 received a second injection of 130 mg of monoclonal antibody 20 months after the first injection, and serum obtained after this second injection was used in a screening test for the presence of anti-idiotype antibody.

Preparation of polyclonal anti-idiotype antibodies

New Zealand white rabbits were injected subcutaneously at multiple sites with 300 µg purified MAb 17-1A emulsified in Freund's complete adjuvant and, 30 days later, boosted intramuscularly with 100 µg of the monoclonal antibody. Sera was collected on day 10 of the secondary response.

Anti-sera was absorbed on immobilized MAb $C_42032$ and MAb 17-1A. The purified monoclonal antibodies (30 mg each) were coupled to 2 ml of Affi-Gel 10 (Bio-Rad Laboratories, Richmond, CA). The anti-sera was then sequentially absorbed on MAb $C_42032$ and MAb 17-1A immunoabsorbents to remove anti-isotypic and anti-idiotypic antibodies, respectively. Absorbed antibodies were eluted with 0.1 M glycine buffer (pH 2.8), immediately neutralized with phosphate buffer, dialyzed against phosphate-buffered saline, and protein quantitated by absorptivity at 280 nm ($E^{1\%}_{280}$=14).

Anti-sera obtained from patients after one injection fo MAb 17-1A was also obtained and purified as described above. Sera from subject No. 23, who received 750 mg of MAb 17-1A, and from subjects No. 08 and 07, who received 133 and 125 mg of MAb 17-1A, respectively, were collected at various times after the first injection of antibody. Samples shown by radioimmunoassay analysis to contain antimurine IgG antibody were sequentially absorbed on MAb $C_42032$ and MAb 17-1A immunoabsorbents to remove anti-isotypic and anti-idiotypic antibodies, respectively, as described above. The anti-idiotype antibody isolated from the sera was shown to be human immunoglobulins by binding to $^{125}$I-labelled anti-human $F(ab')_2$ fragments. The yield of anti-idiotype protein from serum samples varied: 13 µg/ml from No. 08; 8.9 µg/ml from No. 07; and 43 µg/ml from No. 23. The largest amount was obtained from No. 23 serum which also showed the highest levels of anti-mouse globulin antibodies.

Screening sera for presence of anti-idiotype antibodies

To screen serum samples for the presence of anti-idiotype antibody, a competition assay was performed using the rabbit-anti-idiotype antibody and four human sera pre-incubated with $^{125}$I-labelled MAb 17-1A.

Polystyrene beads of 1/4-inch (6.35 mm) size (Precision Plastic Ball Co., Chicago, IL) were washed three times with 95% ethynol. The air-dried beads were incubated with a dilution of either rabbit or human anti-idiotype antibody in 0.02 M sodium tetraborate, pH 8.2. After overnight incubation at 4°C with gentle shaking, the beads were washed three times with phosphate-buffered saline and then incubated for at least three hours at room temperature with phosphate-buffered saline containing 2% bovine serum albumin and 0.04% $NaN_3$. The beads were then exposed to $^{125}$I-labelled MAb 17-1A as the reference idiotype that had been preincubated with the potential source of human anti-idiotype antibody, *i.e.,* human sera was diluted to 25% concentration in phosphate-buffered saline without $Ca^{++}$ and $Mg^{++}$ and supplemented with 2% bovine serum albumin and 0.04% $NaN_3$. After an additional overnight incubation, the beads were washed and the radioactivity bound was measured in a gamma counter.

Three of the sera obtained after one injection with monoclonal antibody, (Nos. 23, 09, and 14) showed inhibition of binding of MAb 17-1A that was higher than that of pre-monoclonal antibody injection samples. Binding inhibition values obtained for post-monoclonal antibody serum of patient No. 14 were low as compared to the other two sera, but higher than that for the pre-monoclonal antibody exposure serum of the same subject. Inhibition values for serum obtained from patient No. 08 seven days after he received a second injection of monoclonal antibody were already high.

Competition assay for detection of anti-idiotype

A competition assay was conducted in a manner similar to that described above to determine the binding of isolated anti-idiotype antibodies to MAb 17-1A, as well as to monoclonal antibodies $C_42032$, $C_41472$ and A5C3.

The results indicated that the binding of anti-idiotype antibodies from three sera (Nos. 08, 07 and 23) to Mab 17-1A was significantly higher than to the three other monoclonal antibodies, two of which ($C_42032$ and $C_41472$) also detect antigenic sites on colorectal carcinoma cells. These sites, however, are different from the sites recognized by MAb 17-1A. The immunoglobulin isolated from sera of all three subjects prior to exposure to MAb 17-1A was concentrated to approximately 2.5 μg/ml and coupled to polystyrene beads. These preparations, however, did not bind any of the monoclonal antibodies tested, indicating the absence of anti-idiotype antibodies in pre-exposure serum.

Cross-reactivity between human anti-idiotype antibodies

A competition assay was conducted to determine whether there was cross-reactivity among the human anti-Id sera.

Results of the competition assay, shown below, indicates significant cross-reactivity between the anti-idiotype sera of patients No. 07 and 23, and slightly less (but still significant) cross-reactivity between the anti-idiotype sera of patients Nos. 08 and 23. Similar cross-reactivity was found between the anti-idiotype sera of patient No. 07 and post-monoclonal antibody serum obtained from patient No. 08 (results not shown). These results indicate that the anti-idiotype antibodies produced by different patients are directed against the same antigenic site.

| 1st Antibody | 2nd Antibody serum of: | | cmp Bound | % of Inhibition of 17-1A MAb binding |
|---|---|---|---|---|
| | None | | 4297 | . |
| Anti-Id 23 | 07 | Pre | 4595 | 0 |
| | | Post | 1231 | 71 |
| | 08 | Pre | 4097 | 5 |
| | | Post | 2585 | 40 |

Epitope detected by anti-idiotype antibody

Hapten inhabition of binding of human anti-idiotype antibodies to MAb 17-1A was performed to show that the anti-idiotype antibodies were directed to the parotope of the idiotype antibody.

A 3 M KCl extract of SW 1222 cells, a cell line derived from colorectal cancer, was prepared as described in Herlyn et al., (1982) *J. Clin. Immunol.* 2: 135-140. The preparation bound MAb 17-1A, indicating that the material contained the antigen in its soluble form. [125]Labelled MAb 17-1A was incubated with the CRC cell extract and with a 3 M KCl extract of melanoma cells which do not bind MAb 17-1A. The antibody-antigen mixtures were then added to beads coated with anti-idiotype antibody obtained from patient No. 23 and the binding compared to the binding of the radiolabelled monoclonal antibody alone. These experiments were performed with non-saturating amounts of iodinated monoclonal antibody in order to detect changes in binding with small amounts of competitive haptens. For control purposes, the iodinated MAb 17-1A was mixed with an extract from melanoma cells that was known not to bind MAb 17-1A. The CRC cell extracts in concentrations of 0.1 or 0.5 mg/ml were found to inhibit the binding of the anti-idiotype antibody from patient No. 23 to iodinated MAb 17-1A by 39% and 68%, respectively. The extract from melanoma cells in concentrations up to 0.5 mg/ml did not significantly affect the monoclonal antibody binding.

This hapten inhibition of the binding reaction indicates the presence of an "internal image" of the CRC epitope on the anti-idiotype antibody. This is also supported by the fact that the finding that the extract of CRC cells did not bind to the anti-idiotype antibodies but did bind, as expected, to MAb 17-1A.

Production of anti-idiotype and anti-(anti-idiotype) antibodies by Human B lymphocytes

Buffy coat cells were obtained from patient Nos. 08 and 23, twenty and five months, respectively, after injection with MAb 17-1A. The cells were stimulated with 10 ng/ml F(ab')$_2$ fragments of 17-1A *in vitro* as described in DeFreitas et al., (1982), *Proc. Natl. Acad. Sci. U.S.A. 79:* 6646-6650. During the following seven days, aliquots of cells were separated into T and B cell populations by rosetting with sheep erythrocytes treated with 2-amino ethylisouronium bromide. *See.* Pellogrino et al., (1975) *Clin. Immunol. & Immunopathol. 3:* 324-333. Both cell populations were stained with F(ab')$_2$ fragments of 17-1A or anti-influenza monoclonal antibody, and goat anti-mouse Ig-FITC. The cell populations were then subsequently analyzed in a cytofluorograph. In addition, peripheral blood mononuclear cells from the same patient were stimulated with F(ab')$_2$ fragments of 17-1A or anti-influenza monoclonal antibodies for nine days in a modified Mishell-Dutton culture for specific human Ig production as described in DeFreitas et al., *supra.* Supernatants from these cultures were assayed in a solid-phase enzyme-linked immunoabsorbent assay for specific human IgG (KPL Laboratories, Gaithersburg, MD).

The percentage of lymphocytes that initially bound 17-1A F(ab')$_2$ of patient No. 08 was 1.2%, and of patient No. 23 was 0.2%. During seven days in culture with MAb 17-1A, the percentage of lymphocytes of patient No. 23 that specifically bound 17-1A F(ab')$_2$ increased from 0.2 to 13%. All the 17-1A binding cells were present in the B cell population. In addition, after nine days, human anti-MAb 17-1A IgG was detected. Incubation of lymphocytes from the same patient with anti-influenza monoclonal antibody under identical conditions produce no detectable human I$_g$ to either MAb 17-1A or anti-influenza monoclonal antibodies.

In another experiment, human B lymphocytes were stimulated to produce anti-(anti-idiotype) antibody. B lymphocytes were collected and stimulated *in vitro* as described above, except that the cells were stimulated with autologous anti-idiotype antibody rather than idiotype antibody. Anti-(anti-idiotype) antibodies were produced by stimulated B lymphocytes and these anti-(anti-Id) Ab were shown to identify as eptiope on CRC cell extract and on whole cells. Thus, the human immune system will produce anti-tumor antibodies in response to stimulation with anti-idiotype antibodies.

Immortal B lymphocytes producing anti-idiotype antibody

Various methods of producing immortal B lymphocytes secreting monoclonal antibodies are known in the art. *See* Kozbor et al., (1983) *Immunology Today 4:* 72-79. Human B lymphocytes secreting anti-(anti-idiotype) antibody, obtained from peripheral blood lymphocytes as described above, can be immortalized, therefore, by one of skill in the art.

One method that can be readily employed is immortalization with Epstein-barr virus (EBV). In this method, the normal lymphocytes described above are infected with EBV *in vitro* and immortal cell lines then establish, for example, by limiting dilution on a feeder layer. *See, e.g.,* Kozbor, et al., (1983), *supra,* and references 51-60 cited therein.

Another approach is to fuse either the above described anti-Id Ab secreting lymphocytes or an EBV-transformed lymphocyte with a human plasmacytoma or lymphoblastoid fusion partner. For example, an EBV-transformed B lymphocyte secreting anti-Id Ab can be fused with, for example, the human lymphoblastoid cell line KR-4. The desired hybridomas would then be selected for in hypoxanthine-aminopterin-thymidine medium containing ouabain, which eliminates the parental cells. Hybridomas are tested for specific antibody production. Positive hybrids are then cloned, recloned and then propagated in bulk culture or in the peritoneal cavity of an immune-suppressed mammal (*e.g.,* nude mouse). *See, e.g.,* Kozbor et al., (1982) *Proc. Natl. Acade. Sci. U.S.A. 79:* 6651-6655.

The above example is presented for illustrative purposes only and is not intended to limit the invention which is defined solely by the claims.

**Claims**

**1.** 1. An immortalised B-lymphocyte that produces a monoclonal, anti-idiotype antibody, said anti-idiotypic antibody bearing an internal image of an epitope present on a solid tumor cell and being for use in the therapy of solid tumors, wherein said anti-idiotypic antibody:

(a) immunoreacts through its paratope with a monoclonal antibody specific for said solid tumour cell epitope; and

(b) will induce formation of anti-anti-idiotypic antibodies specific for said solid tumor cell epitope upon administration to a human.

EP 0 141 783 B2

2.  A monoclonal anti-idiotypic antibody for use in the therapy of solid tumors, said anti-idiotypic antibody bearing an internal image of an epitope present on a solid tumor cell, wherein said anti-idiotypic antibody:
    (a) immunoreacts through its paratope with a monoclonal antibody specific for said solid tumor cell epitope; and
    (b) will induce formation of anti-anti-idiotype antibodies specific for said solid tumor cell epitope upon administration to a human.

3.  A polyclonal preparation for use in the therapy of solid tumors and comprising anti-idiotypic antibodies which bear an internal image of an epitope present on a solid tumor cell, wherein said anti-idiotypic antibodies;
    (a) immunoreact through their paratopes with a paratope of a monoclonal antibody specific for said solid tumor cell epitopes; and
    (b) will induce formation of anti-anti-idiotypic antibodies specific for said solid tumor cell epitope upon administration to a human; and
    (c) are substantially free of anti-isotypic antibodies, said anti-idiotypic antibodies made by the process of immunizing an animal with a monoclonal antitumor antibody.


**Patentansprüche**

1.  Immortalisierter B-Lymphocyt, der einen monoklonalen, anti-idiotypischen Antikörper produziert, wobei der anti-idiotypische Antikörper ein internes Bild eines auf einer festen Tumorzelle vorhandenen Epitops trägt und für die Therapie von festen Tumoren verwendet wird, wobei dieser anti-idiotypische Antikörper
    (a) mittels seines Paratops mit einem für dieses Epitop der festen Tumorzelle spezifischen, monoklonalen Antikörper immunreagiert; und
    (b) nach Verabreichung an Menschen die Bildung von anti-anti-idiotypischen, für dieses Epitop der festen Tumorzelle spezifischen Antikörpern induziert.

2.  Monoklonaler, anti-idiotypischer Antikörper für die Verwendung bei der Therapie von festen Tumoren, wobei der anti-idiotypische Antikörper ein internes Bild eines auf einer festen Tumorzelle vorhandenen Epitops trägt und wobei dieser anti-idiotypische Antikörper
    (a) mittels seines Paratops mit einem für dieses Epitop der festen Tumorzelle spezifischen, monoklonalen Antikörper immunreagiert; und
    (b) nach Verabreichung an Menschen die Bildung von anti-anti-idiotypischen, für dieses Epitop der festen Tumorzelle spezifischen Antikörpern induziert.

3.  Polyklonale Zubereitung für die Verwendung bei der Therapie von festen Tumoren, die anti-idiotypische Antikörper enthält, die ein internes Bild eines auf einer festen Tumorzelle vorhandenen Epitops tragen, wobei diese anti-idiotypischen Antikörper
    (a) mittels ihrer Paratope mit einem Paratop eines für diese Epitope der festen Tumorzellen spezifischen, monoklonalen Antikörpers immunreagieren; und
    (b) nach Verabreichung an Menschen die Bildung von anti-anti-idiotypischen, für dieses Epitop der festen Tumorzelle spezifischen Antikörpern induzieren; und
    (c) im wesentlichen frei sind von anti-isotypischen Antikörpern, wobei die anti-idiotypischen Antikörper mittels des Verfahrens der Immunisierung eines Tieres mit einem monoklonalen Antitumor-Antikörper hergestellt werden.


**Revendications**

1.  Lymphocyte B immortalisé qui produit un anticorps monoclonal anti-idiotypique, ledit anticorps anti-idiotypique portant l'image interne d'un épitope présent sur une cellule de tumeur solide et étant utilisé pour la thérapie des tumeurs solides, dans laquelle ledit anticorps anti-idiotypique :
    (a) immuno-réagit par son paratope avec l'anticorps monoclonal spécifique de l'épitope de cellule de tumeur solide; et
    (b) induira, lors de son administration à l'homme, la formation d'anticorps anti-anti-idiotypiques spécifiques de l'épitope de cellule de tumeur solide.

9

2. Anticorps monoclonal anti-idiotypique pour l'utilisation en thérapie des tumeurs solides, ledit anticorps anti-idiotypique portant l'image interne d'un épitope présent sur une cellule de tumeur solide, dans laquelle ledit anticorps anti-idiotypique :

(a) immuno-réagit par son paratope avec l'anticorps monoclonal spécifique de l'épitope de cellule de tumeur solide; et

(b) induira, lors de son administration à l'homme, la formation d'anticorps anti-anti-idiotypiques spécifiques de l'épitope de cellule de tumeur solide.

3. Préparation polyclonale pour l'utilisation en thérapie de tumeurs solides et comprenant des anticorps anti-idiotypiques qui portent l'image interne d'un épitope présent sur une cellule de tumeur solide, dans laquelle lesdits anticorps anti-idiotypiques :

(a) immuno-réagissent par leurs paratopes avec le paratope d'un anticorps monoclonal spécifique des épitopes de cellule de tumeur solide; et

(b) induiront, lors de leur administration à l'homme, la formation d'anticorps anti-anti-idiotypiques spécifiques d'épitopes de cellule de tumeur solide; et

(c) sont en grande partie exempts d'anticorps anti-isotypiques, lesdits anticorps anti-idiotypiques étant obtenus par l'immunisation d'un animal avec un anticorps monoclonal antitumoral.